# EUROPÄISCHE PATENTSCHRIFT

(11) **EP 3 791 813 B1**
(45) Veröffentlichungstag und Bekanntmachung des Hinweises auf die Patenterteilung: **25.03.2026**
(21) Anmeldenummer: 20195008.6
(22) Anmeldetag: 08.09.2020
(51) Int. Cl.: A61B 17/17

(54) **MEDIZINTECHNISCHE VORRICHTUNG ZUM BESTIMMEN VON POSITION UND ORIENTIERUNG EINER BOHRUNG FÜR EIN IN EINE VERRIEGELUNGSÖFFNUNG EINER ENDOPROTHESE EINZUBRINGENDES VERRIEGELUNGSMITTEL**
MEDICAL DEVICE FOR DETERMINING THE POSITION AND ORIENTATION OF A BORE FOR A LOCKING MEANS TO BE INSERTED INTO A LOCKING OPENING OF AN ENDOPROSTHESIS
DISPOSITIF MÉDICAL PERMETTANT DE DÉTERMINER LA POSITION ET L'ORIENTATION D'UN TROU POUR UN MOYEN DE VERROUILLAGE À INSÉRER DANS UNE OUVERTURE DE VERROUILLAGE D'UNE ENDOPROTHÈSE

(30) Priorität: 16.09.2019 DE 102019124892
(43) Veröffentlichungstag der Anmeldung: 17.03.2021
(73) Patentinhaber: Aesculap AG, 78532 Tuttlingen (DE)
(72) Erfinder: UTZ, Michael, 78532 Tuttlingen (DE)
(74) Vertreter: Winter, Brandl - Partnerschaft mbB

(56) Entgegenhaltungen:
- US-A1- 2015 080 740
- US-A1- 2018 214 240

## Beschreibung

### Technisches Gebiet

Die vorliegende Erfindung betrifft eine medizintechnische Vorrichtung zum Bestimmen von Position und Orientierung einer in einen Knochen einzubringenden Bohrung relativ zu einer Verriegelungsöffnung einer in den Knochen eingebrachten Endoprothese, insbesondere eines Marknagels oder eines Revisionshüftschafts.

Im Rahmen einer Implantierung einer Endoprothese, wie zum Beispiel eines Revisions-Hüftschafts oder eines Marknagels, wird zunächst die Prothese in den Röhrenknochen eingebracht und nach korrekter Positionierung mit Hilfe von Verriegelungsmitteln, welche die dazu bestimmten Verriegelungsöffnungen in der Prothese durchgreifen, im Knochen befestigt. Dafür ist es erforderlich, dass nach dem Einsetzen der Prothese Bohrungen für die Verriegelungsmittel an passender Stelle in den Knochen eingebracht werden. Anschließend kann eine Verriegelungsschraube als Verriegelungsmittel durch den Knochen und die Prothese in die gegenüberliegende Kortikalis des Knochens eingebracht werden, um so die Prothese im Knochen zu verriegeln und gegen Verdrehen im Knochen zu sichern. Gleichzeitig wird außerdem ein korrekter Lastabtrag und gegebenenfalls eine Fixierung von Frakturfragmenten gewährleistet.

Das Auffinden der Verriegelungsöffnungen der Prothese sowie die entsprechende Positionierung und Orientierung dazu passender Bohrungen im Knochen sind im Rahmen einer Implantation in der Regel nicht einfach. Grund dafür ist, dass die Prothese zuerst in den Knochen eingebracht wird und Knochenbohrungen bei dann verdeckter Prothese einzubringen sind, ohne dass der Operateur Kenntnis von der genauen Position und Orientierung der Verriegelungsöffnungen der Prothese im Knochen besitzt. Die Verriegelungsöffnungen sind für ihn nicht sichtbar und müssen dennoch äußerst genau von außen anvisiert werden, da es für einen festen Halt der Prothese und eine möglichst geringe Beeinträchtigung des Patienten wichtig ist, dass der umliegende Knochen möglichst genau an der Position und in der Achse der Verriegelungsöffnungen der Prothese durchbohrt wird.

Es ist bekannt, die Position und Ausrichtung von Verriegelungsöffnungen einer Endoprothese mit Hilfe von abbildenden Diagnoseverfahren wie röntgenologischer Bildgebung zu erfassen und anzuzeigen. Dabei werden sogenannte Spickdrähte unter Verwendung eines Bildwandlers gesetzt. Die Bohrungen in den Knochen werden dann mit kanülierten Bohrern eingebracht. Je nach Geschick und Übung des Arztes dauert eine Präparation unter Bildgebung in der Regel lediglich wenige Minuten und es sind nur wenige Bilder erforderlich. Allerdings können bei Komplikationen oder komplexeren Situationen deutlich mehr Zeit und eine größere Anzahl an Bildern erforderlich sein, was in nachteiliger Weise mit einer erheblichen Strahlenbelastung für den Patienten und den Operateur verbunden ist.

Außerdem ist es bekannt, die Position und Ausrichtung von Verriegelungsöffnungen einer Endoprothese mit einem mechanischen Zielgerät zu erfassen. Dazu werden in der Regel von den Implantatherstellern mechanische Zielgeräte bereitgestellt, die am Implantat zu fixieren sind. Mit reichlich Abstand zu den Weichteilen des Patienten werden dann Position und Orientierung der Verriegelungsbohrungen angezeigt und eine Führung von Werkzeugen, wie Bohrer, ermöglicht. Nachteil derartiger Zielgeräte ist häufig eine durch den großen Abstand zwischen Implantat und Zielgerät sowie die große Länge des Instruments begründete Ungenauigkeit. Auch bedürfen solche mechanischen Zielgeräte einer aufwendigen Wiederaufbereitung und sind im praktischen Einsatz unhandlich, sperrig und groß. Zudem sind sie in der Herstellung sehr aufwändig und teuer.

Aus der US 2015 / 0 080 740 A1 ist bekannt, dass voneinander beabstandete Laser, die nicht parallele Laserstrahlen emittieren, eine Schnittlinie der Laserstrahlen aufweisen. Die Schnittlinie kann eine Bewegungsbahn eines medizinischen/ operativen Instrumentes visualisieren.

Die US 2018 / 0 214 240 A1 offenbart eine Vorrichtung zum Erfassen der Position von Verriegelungsöffnungen an einem Marknagel. Dazu weist die Vorrichtung eine optische Visiereinheit auf. Die optische Visiereinheit weist zwei voneinander beabstandete Linienlaser auf, die jeweils gerade Laserstrahlen emittieren. Die Laserstrahlen sind nicht parallel und derart zueinander ausgerichtet, dass sich die Laserstrahlen treffen und ihr gemeinsamer Schnittpunkt die Bohrachse markiert. Da der Marknagel und die optische Visiereinheit jedoch mit einem Verbindungsarm verbunden sind, hat die Vorrichtung einen hohen Platzbedarf und ist nicht kompakt ausgeführt. Ferner zeigt die Vorrichtung der US 2018 / 0 214 240 A1 lediglich die Position der Verriegelungsöffnungen an dem Marknagel an. Die Vorrichtung ist nicht geeignet, eine Orientierung, insbesondere eine Orientierung einer Bohrachse, mit der in die Verriegelungsöffnungen gebohrt werden soll, um den Marknagel am Knochen zu fixieren, anzuzeigen.

Vor diesem Hintergrund liegt der vorliegenden Erfindung die Aufgabe zugrunde, die genannten Nachteile des Stands der Technik zu verringern, insbesondere eine Vorrichtung zu schaffen, mit der ein Ersetzen bekannter Zielgeräte unter Beibehaltung oder Verbesserung der Zielgenauigkeit möglich ist. Außerdem soll mittels der Erfindung ein Ersatz für die Bildgebung mittels Roentgen geschaffen werden, so dass die Strahlenbelastung von medizinischem Personal und Patient verringert wird.

Diese Aufgabe wird nach der vorliegenden Erfindung gelöst durch eine Vorrichtung nach Anspruch 1.

Genauer ausgedrückt wird eine medizintechnische Vorrichtung zum Bestimmen von Position und/oder Orientierung einer in einen Knochen einzubringenden Bohrung, auch als Knochenbohrung bezeichnet, relativ zu einer Verriegelungsöffnung einer in den Knochen eingebrachten Endoprothese, insbesondere eines Marknagels oder eines Revisionshüftschafts, bereitgestellt, welche Vorrichtung eine Koppelstruktur zum lagebestimmten mittelbaren oder unmittelbaren Koppeln der Vorrichtung an der Endoprothese und eine optische Visiereinrichtung zum Anzeigen der Position und/oder Orientierung der Bohrung, insbesondere einer Bohrachse der Bohrung, aufweist. Die optische Visiereinrichtung weist wenigstens zwei voneinander bebstandete Linienlaser auf, die zum Ausstrahlen zumindest zweier nichtparalleler Laserstrahlen eingerichtet sind. Die Linienlaser sind derart zueinander ausgerichtet, dass ihre gemeinsame Schnittlinie die Bohrachse mittelbar oder unmittelbar markiert. Einer der zumindest zwei nichtparallelen Laserstrahlen erstreckt sich entlang einer Längsachse der Endoprothese.

Durch die Verwendung von Laserstrahlen bzw. Linienlaserstrahlen können dem Operateur in besonders einfacher und übersichtlicher Weise Informationen über die Position und Ausrichtung der Verriegelungsöffnungen und der in den Knochen einzubringenden Knochenbohrungen vermittelt werden.

Lagebestimmt gekoppelt im Sinne der Erfindung bedeutet insbesondere, dass die Vorrichtung bestimmt, hinsichtlich ihrer Position und Orientierung relativ zu der in den Knochen eingebrachten Endoprothese, positioniert und gehalten ist. Die Verriegelungsöffnung und die in den Knochen einzubringende Knochenbohrung dienen der Aufnahme eines die Endoprothese mit dem Knochen verriegelnden Verriegelungsmittels.

Es ist ein besonderer Vorteil der Erfindung, dass die Vorrichtung durch Nutzung einer optischen Visiereinrichtung einen besonders kleinen Platzbedarf, nur wenige für eine Verschmutzung anfällige Teile und eine im Wesentlichen größenunabhängige Genauigkeit besitzt. Sie ist daher besonders genau, handlich, nicht sperrig, verlässlich und günstig, sowohl im Rahmen ihrer Verwendung als auch im Rahmen der Aufbereitung. Die optische Visiereinrichtung ist dabei als Single-Use Instrument oder als Multiple-Use Instrument realisierbar. Die Erfindung stellt quasi ein Zielgerät oder eine Markierungsvorrichtung zur Verfügung, das in einfacher Weise unmittelbar am Implantat und/ oder an einem damit lagedefiniert verbundenen Operationsbesteck und/ oder Operationsinstrument anzubringen ist. Die Positionierung und/oder Orientierung von Knochenbohrungen mittels der Vorrichtung nach der Erfindung bietet gegenüber den aus dem Stand der Technik bekannten Möglichkeiten eine höhere Genauigkeit unter Vermeidung von Strahlenbelastung und gleichzeitiger Kostenersparnis. Zudem ist die optische Visiereinrichtung so ausgestaltet, dass Parallaxenfehler vermieden werden.

Mit Hilfe der Erfindung können bei einer Implantierung einer Endoprothese die zu deren bestimmungsgemäßer Verankerung und Befestigung im Knochen erforderlichen Knochenbohrungen besonders einfach und korrekt in den Knochen eingebracht werden. Dies ist bedingt durch eine direkte, robuste und genaue Anzeige der Position und/oder Orientierung der Verriegelungsöffnungen der Prothese und damit gleichzeitig der Position und/oder Orientierung der in den Knochen einzubringenden Knochenbohrungen und/oder deren Bohrungsachsen. Die Anzeige der Position und Orientierung kann dabei in besonders vorteilhafter Weise auch nach Einbringen / Einsetzen der Prothese an passender Stelle in den Knochen erfolgen, also bei genau bestimmungsgemäß sitzender Prothese. Nach dem Ausführen der Knochenbohrung(en) kann abermals ohne Veränderung der Lage der Prothese im Knochen eine Verriegelungsschraube durch den Knochen und die Prothese in die gegenüberliegende Kortikalis des Knochens eingebracht werden, um diese so im Knochen zu verriegeln.

Das Auffinden von Verriegelungsöffnungen der Prothese sowie die Positionierung und Orientierung dazu passender Bohrungen im Knochen erfolgt durch die Erfindung in besonders einfacher und anwenderfreundlichen Weise und ist im Rahmen einer Implantation besonders einfach und fehlersicher, da die Prothese zuerst in die bestimmungsgemäße Lage in dem Knochen gebracht wird und die Positions- /Orientierungsbestimmung der Knochenbohrung ohne mechanische Belastung der dann verdeckten Prothese möglich ist.

Vorteilhafte Ausführungsformen der Erfindung sind in den Unteransprüchen beansprucht und werden nachfolgend näher erläutert.

Eine Ausführungsform ist dadurch gekennzeichnet, dass die optische Visiereinrichtung zumindest eine Lasereinrichtung/ einen Laser aufweist. Die Lasereinrichtung weist dabei vorzugsweise wenigstens eine Lichtquelle auf, die wenigstens einen Laserstrahl generiert, und wenigstens einen Laserscanner, der den wenigstens einen Laserstrahl rasterförmig oder linienförmig ablenkt. Der Laserscanner weißt dabei vorzugsweise einen Scankopf und eine Treiber- und Ansteuerelektronik auf. Im Scankopf wird der Laserstrahl abgelenkt, dessen Ablenkungswinkel gemessen und elektronisch geregelt wird. Vorzugsweise ist der Scankopf einen Spiegelscanner. Alternativ oder zusätzlich zu dem Laserscanner können diffraktive optische Elemente vorzugsweise in Kombination mit Powell-Linsen zur Generierung von Linien oder Rastern verwendet werden.

Nach einer weiteren Ausführungsform kann der Laser insbesondere als Linienlaser ausgebildet sein. Darunter ist ein Laser mit einer speziellen Optik zu verstehen, mit der beim Auftreffen des Laserstrahls auf ein Objekt anstatt eines Punktes eine Linie erzeugt wird. Dies kann zum Beispiel durch eine eindimensionale optische Aufweitung des Laserstrahls (z. B. durch diffraktive optische Elemente) oder durch eine schnelle Oszillation (z. B. durch einen Laserscanner) des Laserstrahls erfolgen. Durch Verwendung eines Lasers kann eine besonders genaue Anzeige der Position und Ausrichtung der Bohrungsachse erfolgen. Außerdem benötigt ein Laser nur geringen Bauraum, so dass die Vorrichtung besonders klein ausgebildet werden kann, was ein weites Einsatzgebiet und eine hohe Flexibilität ermöglicht. Das Operationsfeld ist in vorteilhafter Weise nicht von der Vorrichtung verdeckt, sondern für den Operateur nahezu uneingeschränkt frei sichtbar und zugänglich.

Vorzugsweise ist die Visiereinrichtung zum Ausstrahlen zumindest zweier nichtparalleler Laserstrahlen, vorzugsweise Linienlaserstrahlen, eingerichtet. Diese können insbesondere derart zueinander ausgerichtet sein, dass ihr gemeinsamer (Linien-)Schnittpunkt die Bohrachse mittelbar oder unmittelbar markiert, vorzugsweise direkt auf das Verriegelungsmittel oder eine Bohrlehre. **In** einer Variante kann die Visiereinrichtung zum Ausstrahlen zumindest zweier Linienlaserstrahlen eingerichtet sein. Diese können derart zueinander ausgerichtet sein, dass ihre gemeinsame (Linien- )Schnittachse die Bohrachse mittelbar oder unmittelbar markiert. Eine mittelbare Markierung kann erfolgen, indem der Durchmesser des Bohrers in die Erstellung der Markierung einbezogen wird und eine Markierung auf der Umfangsfläche des Bohrers erfolgt. In anderen Worten ausgedrückt weist die optische Visiereinrichtung wenigstens zwei Lasereinrichtungen auf, die voneinander an der Visiereinrichtung örtlich beabstandet sind bzw. weist die optische Visiereinrichtung zwei voneinander örtlich beabstandete transparente Öffnungen (Austrittsfenster) auf, aus denen zwei Linienlaser ausgestrahlt werden. Diese Linienlaser sind nicht parallel zueinander, sondern schneiden sich in einer Schnittlinie.

Diese Schnittlinie ist in einer ersten Ausführungsform senkrecht zu der Öffnungsfläche, genauer zu dem Mittelpunkt der Öffnungsfläche, die von der Verriegelungsöffnung einer in den Knochen eingebrachten Endoprothese aufgespannt wird. Somit bildet die Schnittlinie der beiden Linienlaser die genaue Bohrachse.

Es ist besonders vorteilhaft für die Orientierung des Operateurs, wenn ein erster Laserstrahl der Laserstrahlen bzw. ein erster Linienlaserstrahl der Linienlaserstrahlen senkrecht zu einer Längsachse der Endoprothese ausgerichtet ist, sich also radial von der Längsachse der Endoprothese erstreckt. Dies erleichtert die Orientierung des Operateurs und vermittelt ihm einen grundlegenden Anhaltspunkt für die Lage der Endoprothese relativ zum Körper des Patienten.

Eine einfache und gut erkennbare Markierung der Position und Ausrichtung der Bohrachse kann bewirkt werden, wenn ein zweiter Laserstrahl der Laserstrahlen bzw. ein zweiter Linienlaserstrahl der Linienlaserstrahlen schräg zum ersten Laserstrahl bzw. Linienlaserstrahl ausgerichtet ist und vorzugsweise auch senkrecht zu einer Längsachse der Endoprothese ausgerichtet ist, sich also auch vorzugsweise radial von der Längsachse der Endoprothese erstreckt. Die schräge Ausrichtung bedeutet, dass der erste Laserstrahl und der zweite Laserstrahl einen Winkelaufspannen. Vorzugsweise können sich der erste Laserstrahl / Linienlaserstrahl und der zweite Laserstrahl / Linienlaserstrahl einander in der Bohrachse schneiden. Auf diese Weise kann die Lage und Ausrichtung der Bohrachse für den Operateur unmittelbar kenntlich gemacht werden. Alternativ können sie einander in einem definierten Abstand von der Bohrachse schneiden. Im Rahmen der Erfindung können auch weitere Laserstrahlen, zum Beispiel ein dritter Laserstrahl, verwendet werden, um so eine eindeutige Positions- und Ausrichtungsbestimmung zu bewirken.

In einer zweiten Ausführungsform kann zusätzlich zu der Konfiguration der ersten Ausführungsform ein dritter Laserstrahl bzw. ein dritter Linienlaserstrahl von der optischen Visiereinrichtung ausgesendet werden. Der dritte Linienstrahl ist nicht senkrecht zu einer Längsachse der Endoprothese ausgerichtet, und erstreckt sich nicht radial von der Längsachse der Endoprothese. Der dritte Linienlaser schneidet jedoch den ersten und zweiten Linienstrahl. Der dritte Linienlaser lässt somit ein rotatorisches verdrehen einer Bohrlehre erkennen, in einem Fall in dem zwei Bohrungen hintereinander (entlang der Längsachse der Endoprothese) notwendig sind.

Ein besonders einfaches Einbringen der Knochenbohrung kann mit Hilfe der Erfindung bewirkt werden, wenn die erfindungsgemäße Vorrichtung eine Bohrlehre oder Bohrschablone mit einer Markierung zur lagebestimmten Ausrichtung relativ zu den von der Visiereinrichtung ausgestrahlten Laserstrahlen umfasst. Diese kann mit Hilfe der Laserstrahlen in bestimmungsgemäßer Weise relativ zum Knochen und zur Prothese ausgerichtet werden und eine Zwangsführung des Bohrers bewirken.

Zusammenfassend kann man sagen, dass die Erfindung eine Projektion von Bohrungen / Bohrachsen für Verriegelungsschrauben bei Marknägeln und Revisionshüftschäften mittels mindestens zweier Linienlaser und insbesondere eine medizintechnische Markierungsvorrichtung mit einer optischen Zieleinrichtung zur Verfügung stellt, die anhand zweier Linienlaser zwei Linien entlang der Extremität projizieren kann. Die eine Linie kann dabei genau eine erste Ebene anzeigen, in der sich auch das Implantat und auch die Achsen mindestens einer Verriegelungsbohrung befinden. Der zweite Linienlaser kann insbesondere eine zweite Ebene schräg dazu aufspannen. Diese zweite Ebene kann die erste Ebene entweder genau in der Achse mindestens einer Verriegelungsbohrung oder zumindest unter einem definierten Winkel in einem definierten Abstand zum Implantat schneiden. Eine passende Bohrlehre kann nun anhand der angezeigten Linien oder Ebenen so im Raum ausgerichtet werden, dass sie genau mit der Achse der Verriegelungsbohrung fluchtet. Weicht die Orientierung der Bohrlehre von der gewünschten Ausrichtung ab, kann dies anhand der projizierten Laserlinien / Laserebenen erkannt und korrigiert werden.

Mit Hilfe der Erfindung können gegenüber dem Stand der Technik insbesondere die folgenden Vorteile bewirkt werden:
- Keine Strahlenbelastung
- Höhere Genauigkeit als Zielgeräte
- Reduzierte Aufbereitungs- und Herstellkosten

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung.

### Kurzbeschreibung der Figuren

Dabei zeigen:
Fig. 1 eine perspektivische Ansicht einer Ausführungsform einer Vorrichtung nach der Erfindung mit angedeuteten Linienlaserstrahlen,
Fig. 2 eine perspektivische Ansicht der Ausführungsform der Figur 1 aus einer anderen Blickrichtung,
Fig. 3 eine perspektivische Ansicht der Ausführungsform der Figur 1 aus einer anderen Blickrichtung,
Fig. 4 eine perspektivische Ansicht der Ausführungsform der Vorrichtung gekoppelt an ein Instrument zur Handhabung einer Endoprothese,
Fig. 5 die Zusammenstellung der Fig. 4 aus einer anderen Blickrichtung,
Fig. 6 die Zusammenstellung der Fig. 4 aus einer anderen Blickrichtung,
Fig. 7 einen vergrößerten Ausschnitt der Fig. 5,
Fig. 8 einen vergrößerten Ausschnitt der Fig. 6,
Fig. 9 eine Ansicht der Fig. 8 aus der gegenüberliegenden Blickrichtung und
Fig. 10 einen vergrößerten Ausschnitt der Fig. 4.

### Beschreibung der Ausführungsbeispiele

Nachstehend werden Ausführungsbeispiele der vorliegenden Offenbarung auf der Basis der zugehörigen Figuren beschrieben.

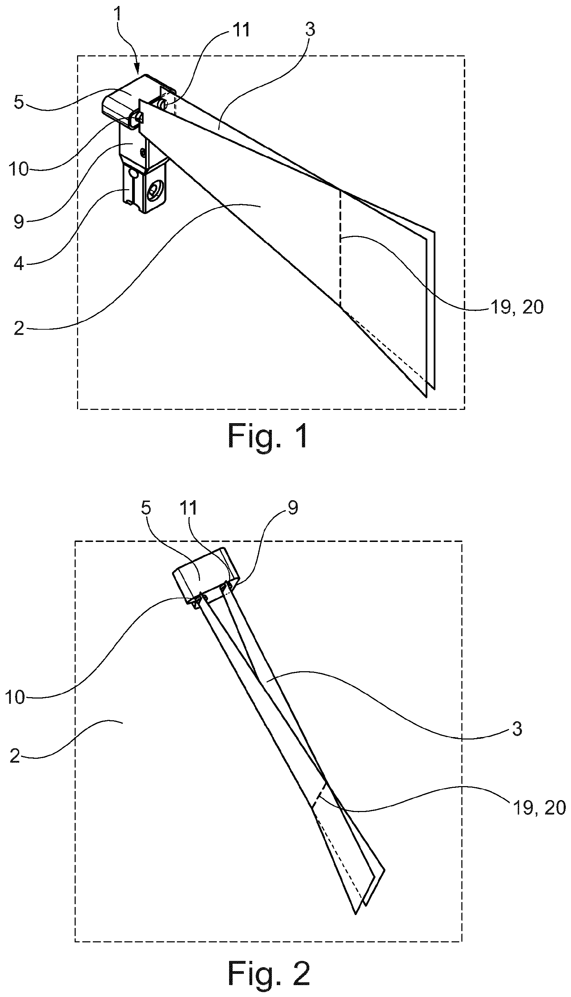

Die Figuren 1 bis 3 zeigen eine Ausführungsform einer Vorrichtung 1 nach der Erfindung mit angedeuteten Laserstrahlen 2, 3 hier in Form eines ersten Linienlaserstrahls 2 und eines zweiten Linienlaserstrahls 3.

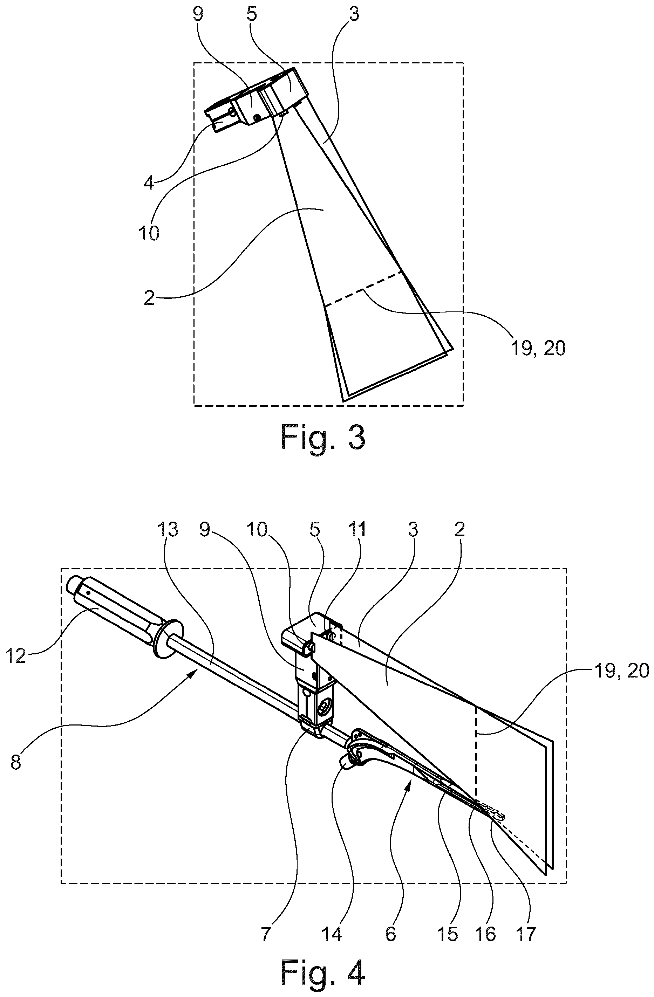

Die Vorrichtung 1 weist eine Koppelstruktur 4 und eine daran angeordnete optische Visiereinrichtung 5 auf. Die Koppelstruktur 4 dient einem lagebestimmten mittelbaren oder unmittelbaren Koppeln der Vorrichtung 1 an einer in den Figuren 1 bis 3 nicht dargestellten Endoprothese 6, die in den Figuren 4 bis 10 dargestellt ist. Mittels der optischen Visiereinrichtung 4 werden eine Position und eine Orientierung einer Bohrachse einer in einen Knochen zur Befestigung der Endoprothese einzubringenden Bohrung angezeigt.

Die Koppelstruktur 4 umfasst ein Klemmelement 7, mit dem die Vorrichtung 1 an einem in den Figuren 4 bis 10 dargestellten Handhabungsinstrument 8 für die Endoprothese 6 lagebestimmt koppelbar ist. Auf der dem Klemmelement 7 gegenüberliegenden Seite ist ein Gehäuse 9 ausgebildet, in dem ein in den Figuren nicht dargestellter Laser sowie eine Optik zur Strahlformung und Strahlführung gegenüber der Umgebung abgedichtet aufgenommen sind. In dem Gehäuse 9 ist ein erstes Austrittsfenster 10 für den ersten Laserstrahl 2 und ein davon beabstandetes zweites Austrittsfenster 11 für den zweiten Laserstrahl 3 ausgebildet.

Die Figuren 4 bis 10 zeigen die Vorrichtung 1 gekoppelt mit dem Handhabungsinstrument 8. Dieses weist einen Handgriff 12 und einen daran distal angeordneten Stab 13 auf, an dessen Ende die Endoprothese 6 zur Handhabung und Positionierung im Rahmen einer Implantation derart gehalten ist, dass die Ausrichtung und die Position der Prothese 6 relativ zum Handhabungsinstrument 8 definiert und bekannt sind.

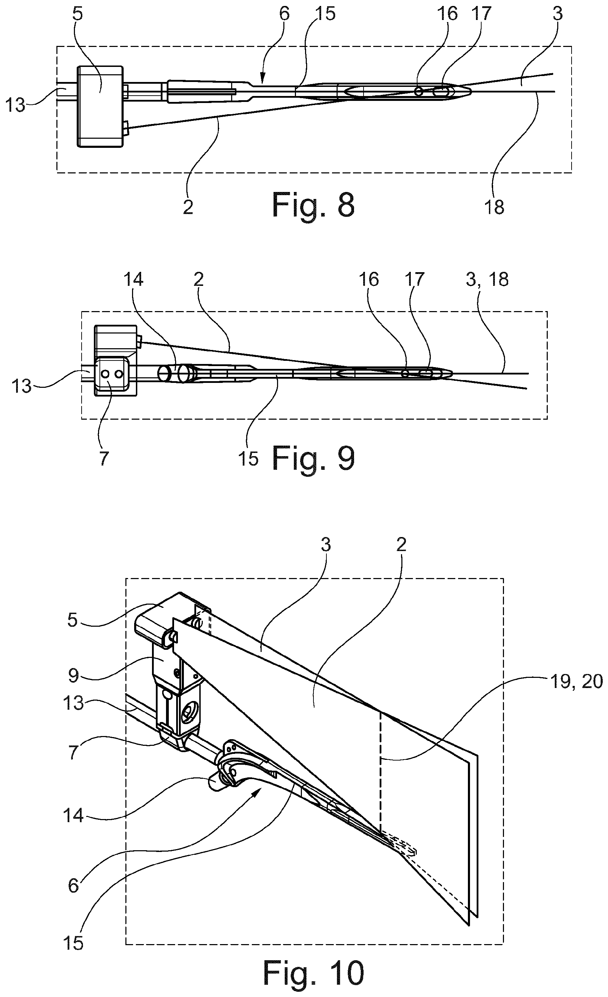

Bei der Endoprothese 6 handelt es sich im vorliegenden Beispiel um einen Revisionshüftschaft 6 mit einer proximal angeordneten Halterung 14 für eine in den Figuren nicht gezeigte Gelenkkugel und einem distalen Schaft 15 zum Einbringen in einen Oberschenkelknochen eines Patienten. Der Revisionshüftschaft 6 wird mittels in den Figuren nicht gezeigten Schrauben im kortikalen Knochengewebe fest verankert. Zu diesem Zweck sind in den Schaft 15 eine erste Verriegelungsöffnung 16 und eine zweite Verriegelungsöffnung 17 eingebracht (besonders gut zu erkennen in Figur 9). Die in den Knochen einzubringenden Bohrungen sind für einen festen Sitz der Endoprothese 6 nach dem Einsetzen derselben in den Knochen in der relativ zu den jeweiligen Verriegelungsöffnungen 16, 17 korrekten Lage und Ausrichtung zu erstellen.

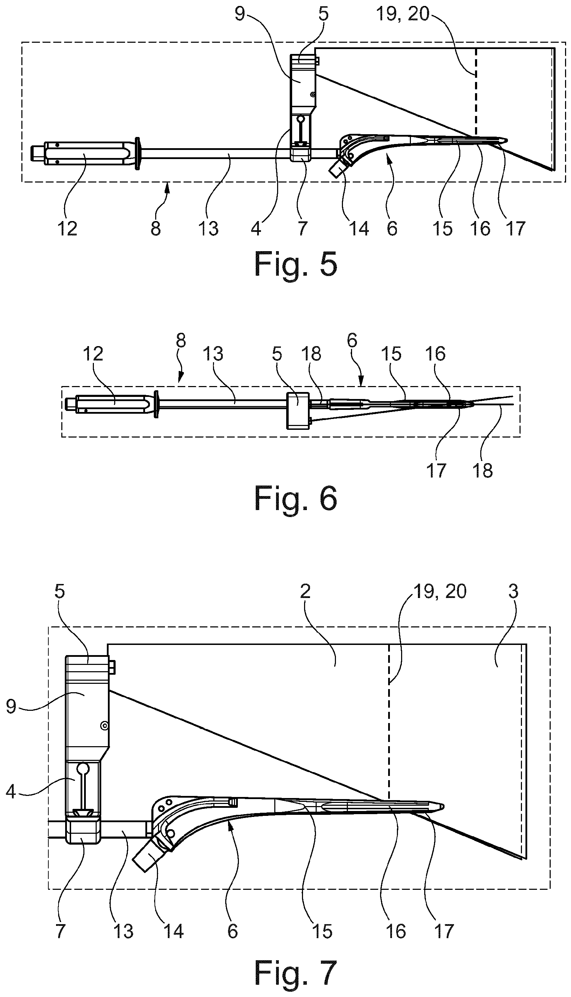

Mittels der von der Vorrichtung 1 ausgestrahlten Laserstrahlen 2, 3 erhält der Operateur eine Orientierung und Anzeige hinsichtlich der Position und der Ausrichtung der Verriegelungsöffnungen 16, 17 der in den Knochen eingebrachten Endoprothese 6. Wie besonders deutlich anhand der Figuren 6, 8 und 9 ersichtlich ist, ist der erste Linienlaserstrahl 2 in Richtung der Längsachse 18 des Revisionshüftschafts 6 sowie in Richtung der Längsachse 19 (in den Figuren gestrichelt dargestellt) der Verriegelungsöffnung 16 ausgerichtet. Anders ausgedrückt liegen die Längsachse 18 der Prothese 6 und auch die Längsachse 19 der Verriegelungsöffnung 16 jeweils in der durch den ersten Linienlaserstrahl 2 definierten Ebene bzw. sind dazusenkrecht. Der zweite Linienlaserstrahl 3 ist relativ zum ersten Linienlaserstrahl 2 nicht parallel und schräg und außerdem ebenfalls in Richtung der Längsachse 19 der Verriegelungsöffnung 16 ausgerichtet, so dass sich der erste Linienlaserstrahl 2 und der zweite Linienlaserstrahl 3 bzw. die durch diese definierten Ebenen in einer gemeinsamen Schnittgeraden 20 (im Falle von zwei normalen Laserstrahlen ist die ein Schnittpunkt) schneiden. Die Schnittgerade 20 ist infolge der Ausrichtung der Laserstrahlen 2, 3 relativ zur Prothese 6 und zur Längsachse 19 der Verriegelungsöffnung 16 senkrecht mit dieser und zeigt dem Operateur damit sowohl die Position als auch die Ausrichtung der Verriegelungsöffnung 16 im Knochen an. Es sei angemerkt, dass die Längsachse 19 der Verriegelungsöffnung 16 und die Bohrachse 19 der zur Verriegelungsöffnung 16 passend in den Knochen einzubringenden Bohrung identisch sind.

Auch wenn die Schnittachse 20 lediglich mit der Längsachse 19 der Verriegelungsöffnung 16 senkrecht ist, ermöglicht die Vorrichtung 1 außerdem eine korrekte Bestimmung der Position und Ausrichtung einer Bohrachse 21 einer für die Verriegelungsöffnung 17 in den Knochen einzubringenden Bohrung, wenn die Position und Ausrichtung der beiden Verriegelungsöffnungen 16, 17 relativ zueinander bekannt sind. Es sei angemerkt, dass die Längsachse 21 der Verriegelungsöffnung 17 und die Bohrachse 21 der zur Verriegelungsöffnung 17 passend in den Knochen einzubringenden Bohrung identisch sind.

Insbesondere kann eine in den Figuren nicht dargestellte Bohrlehre oder Bohrschablone relativ zur Schnittgeraden bzw. zum Schnittpunkt der Laser 2, 3 ausgerichtet werden, so dass die Bohrachse der jeweils in den Knochen einzubringenden Bohrung genau durch die Bohrlehre oder Bohrschablone angezeigt wird.

### Bezugszeichenliste

- 1: Vorrichtung
- 2: Laserstrahl, Linienlaserstrahl, Ebene
- 3: Laserstrahl, Linienlaserstrahl, Ebene
- 4: Koppelstruktur
- 5: Visiereinrichtung
- 6: Endoprothese, Revisionshüftschaft
- 7: Klemmelement
- 8: Handhabungsinstrument
- 9: Gehäuse
- 10: Austrittsfenster
- 11: Austrittsfenster
- 12: Handgriff
- 13: Stab
- 14: Halterung
- 15: Schaft
- 16: Verriegelungsöffnung
- 17: Verriegelungsöffnung
- 18: Längsachse des Schafts 15
- 19: Längsachse, Bohrachse
- 20: Schnittgerade, Schnittpunkt
- 21: Längsachse, Bohrachse

## Patentansprüche

1. Medizintechnische Vorrichtung (1) zum Bestimmen von Position und Orientierung einer Bohrachse (19, 21) einer in einen Knochen einzubringenden Knochenbohrung relativ zu einer Verriegelungsöffnung (16, 17) einer in den Knochen eingebrachten Endoprothese (6), insbesondere eines Marknagels oder eines Revisionshüftschafts (6), für ein die Endoprothese (6) mit dem Knochen verriegelndes Verriegelungsmittel, wobei die medizinische Vorrichtung (1) eine Koppelstruktur (4) zum lagebestimmten mittelbaren oder unmittelbaren Koppeln der Vorrichtung (1) an der Endoprothese (6) und eine optische Visiereinrichtung (5) zum Anzeigen der Position und Orientierung der Bohrachse (19, 21) aufweist, wobei
die optische Visiereinrichtung (5) wenigstens zwei voneinander beabstandete Linienlaser aufweist und zum Ausstrahlen zumindest zweier nichtparalleler Laserstrahlen (2, 3) eingerichtet ist, und
die nichtparallelen Laserstrahlen (2, 3) derart zueinander ausgerichtet sind, dass eine gemeinsame Schnittlinie der nichtparallelen Laserstrahlen (2, 3) die Bohrachse (19, 21) mittelbar oder unmittelbar markiert,
**dadurch gekennzeichnet, dass**
sich einer der zumindest zwei nichtparallelen Laserstrahlen (2, 3) entlang einer Längsachse (18) der Endoprothese (6) erstreckt.

2. Medizintechnische Vorrichtung (1) nach Anspruch 1, **dadurch gekennzeichnet, dass** die optische Visiereinrichtung (5) wenigstens drei Laser, insbesondere Linienlaser, aufweist.

3. Medizintechnische Vorrichtung (1) nach Anspruch 1 oder 2, **dadurch gekennzeichnet, dass** der Laser als Laserscanner ausgebildet ist oder optische Elemente aufweist, die einen Linienlaser erzeugen.

4. Medizintechnische Vorrichtung (1) nach einem der vorstehenden Ansprüche, **dadurch gekennzeichnet, dass** ein erster Laserstrahl (2) bzw. Linienlaserstrahl (2) senkrecht zu der Längsachse (18) der Endoprothese (6) ausgerichtet ist.

5. Medizintechnische Vorrichtung (1) nach Anspruch 4, **dadurch gekennzeichnet, dass** ein zweiter Laserstrahl (3) bzw. Linienlaserstrahl (3) schräg zum ersten Laserstrahl (2) bzw. ersten Linienlaserstrahl (2) ausgerichtet ist.

6. Medizintechnische Vorrichtung (1) nach einem der vorstehenden Ansprüche, **dadurch gekennzeichnet, dass** der erste Laserstrahl (2) / Linienlaserstrahl (2) und der zweite Laserstrahl (3) / Linienlaserstrahl (3) einander in der Bohrachse (19, 21) schneiden oder einander in einem definierten Abstand von der Bohrachse (19, 21) schneiden.

7. Medizintechnische Vorrichtung (1) nach einem der vorstehenden Ansprüche, **dadurch gekennzeichnet, dass** diese eine Bohrlehre oder Bohrschablone mit einer Markierung zur lagebestimmten Ausrichtung relativ zu den von der Visiereinrichtung (5) ausgestrahlten Laserstrahlen (2, 3) umfasst.

## Claims

1. A medical device (1) for determining the position and orientation of a drilling axis (19, 21) of a bone drill to be inserted into a bone relative to a locking opening (16, 17) of an endoprosthesis (6) inserted into the bone, in particular a medullary nail or a revision hip stem (6), for locking means locking the endoprosthesis (6) to the bone, the medical device (1) having a coupling structure (4) for indirectly or directly coupling the device (1) to the endoprosthesis (6) in a position-determined manner and an optical sight (5) for indicating the position and orientation of the drilling axis (19, 21), wherein
the optical sight (5) comprises at least two line lasers spaced apart from one another and is configured to emit at least two non-parallel laser beams (2, 3), and
the non-parallel laser beams (2, 3) are aligned with one another in such a way that a common intersecting line of the non-parallel laser beams (2, 3) marks the drilling axis (19, 21) directly or indirectly, and
**characterized in that**
one of the at least two non-parallel laser beams (2, 3) extends along a longitudinal axis (18) of the endoprosthesis (6).

2. The medical device (1) according to claim 1, **characterized in that** the optical sight (5) has at least three lasers, in particular line lasers.

3. The medical device (1) according to claim 1 or 2, **characterized in that** the laser is configured as a laser scanner or has optical elements that generate a line laser.

4. The medical device (1) according to any of the preceding claims, **characterized in that** a first laser beam (2) resp. line laser beam (2) is aligned perpendicular to the longitudinal axis (18) of the endoprosthesis (6).

5. The medical device (1) according to claim 4, **characterized in that** a second laser beam (3) resp. line laser beam (3) is aligned slantingly to the first laser beam (2) resp. first line laser beam (2).

6. The medical device (1) according to any of the preceding claims, **characterized in that** the first laser beam (2) / line laser beam (2) and the second laser beam (3) / line laser beam (3) intersect each other in the drilling axis (19, 21) or intersect each other at a defined distance from the drilling axis (19, 21).

7. The medical device (1) according to any of the preceding claims, **characterized in that** it comprises a drilling jig or drilling template with a marking for positional alignment relative to the laser beams (2, 3) emitted by the sight (5).

## Revendications

1. Dispositif médical (1) permettant de déterminer la position et l'orientation d'un axe de perçage (19, 21) d'un perçage osseux à réaliser dans un os par rapport à une ouverture de verrouillage (16, 17) d'une endoprothèse (6) insérée dans l'os, en particulier d'un clou intramédullaire ou d'une tige de hanche de révision (6), pour un moyen de verrouillage verrouillant l'endoprothèse (6) à l'os, dans lequel le dispositif médical (1) présente une structure d'accouplement (4) pour accoupler de manière indirecte ou directe le dispositif (1) à l'endoprothèse (6) dans une position déterminée et un appareil de visée optique (5) permettant d'afficher la position et l'orientation de l'axe de perçage (19, 21), dans lequel
l'appareil de visée optique (5) présente au moins deux lasers linéaires espacés l'un de l'autre et est configuré pour émettre au moins deux faisceaux laser non parallèles (2, 3), et
les faisceaux laser non parallèles (2, 3) sont alignés l'un par rapport à l'autre de sorte qu'une ligne d'intersection commune des faisceaux laser non parallèles (2, 3) marque directement ou indirectement l'axe de perçage (19, 21),
**caractérisé en ce que**
l'un des au moins deux rayons laser non parallèles (2, 3) s'étend le long d'un axe longitudinal (18) de l'endoprothèse (6).

2. Dispositif médical (1) selon la revendication 1, **caractérisé en ce que** l'appareil de visée optique (5) présente au moins trois lasers, en particulier des lasers linéaires.

3. Dispositif médical (1) selon la revendication 1 ou 2, **caractérisé en ce que** le laser est conçu en tant que scanner laser ou présente des éléments optiques qui génèrent un laser linéaire.

4. Dispositif médical (1) selon l'une quelconque des revendications précédentes, **caractérisé en ce qu'**un premier faisceau laser (2) ou faisceau laser linéaire (2) est orienté perpendiculairement à l'axe longitudinal (18) de l'endoprothèse (6).

5. Dispositif médical (1) selon la revendication 4, **caractérisé en ce qu'**un second faisceau laser (3) ou faisceau laser linéaire (3) est orienté obliquement par rapport au premier faisceau laser (2) ou premier faisceau laser linéaire (2).

6. Dispositif médical (1) selon l'une quelconque des revendications précédentes, **caractérisé en ce que** le premier faisceau laser (2) / faisceau laser linéaire (2) et le second faisceau laser (3) / faisceau laser linéaire (3) se croisent dans l'axe de perçage (19, 21) ou se croisent à une distance définie de l'axe de perçage (19, 21).

7. Dispositif médical (1) selon l'une quelconque des revendications précédentes, **caractérisé en ce qu'**il comprend un guide ou un gabarit de forage avec un repère pour l'alignement dans une position déterminée par rapport aux faisceaux laser (2, 3) émis par l'appareil de visée (5).
